# EUROPÄISCHE PATENTSCHRIFT

(11) **EP 1 100 780 B1**
(45) Veröffentlichungstag und Bekanntmachung des Hinweises auf die Patenterteilung: **25.04.2007**
(21) Anmeldenummer: 99936584.4
(22) Anmeldetag: 22.07.1999
(51) Int. Cl.: C07D 209/60, A61K 31/40

(54) **2,3,3a,4,9,9a-HEXAHYDRO-8-HYDROXY-1H-BENZ[F]INDOLE, VERFAHREN ZU IHRER HERSTELLUNG UND IHRE VERWENDUNG ALS ARZNEIMITTEL**
2,3,3a,4,9,9a-HEXAHYDRO-8-HYDROXY-1H-BENZ[F]INDOLES, A METHOD FOR THE PRODUCTION THEREOF, AND THEIR USE AS MEDICAMENTS
2,3,3a,4,9,9a-HEXAHYDRO-8-HYDROXY-1H-BENZ[F]INDOLES, LEURS PROCEDES DE PREPARATION ET LEUR UTILISATION COMME MEDICAMENTS

(30) Priorität: 31.07.1998 DE 19834714
(43) Veröffentlichungstag der Anmeldung: 23.05.2001
(73) Patentinhaber: Boehringer Ingelheim Pharma GmbH & Co.KG, 55216 Ingelheim am Rhein (DE)
(72) Erfinder: GRAUERT, Matthias, D-55218 Ingelheim am Rhein (DE); HOENKE, Christoph, D-55218 Ingelheim am Rhein (DE); CARTER, Adrian, D-55411 Bingen am Rhein (DE); BECHTEL, Wolf-Dietrich, D-55437 Appenheim (DE); WEISER, Thomas, D-55268 Nieder-Olm (DE); PALLUK, Rainer, D-55411 Bingen am Rhein (DE); PSCHORN, Uwe, D-55126 Mainz (DE)
(74) Vertreter: Kläs, Heinz-Gerd
(86) Internationale Anmeldenummer: PCT/EP1999/005222
(87) Internationale Veröffentlichungsnummer: WO 2000/007986

(56) Entgegenhaltungen:
- WO-A-91/00856
- WO-A-92/21654
- WO-A-97/47602
- CHEMICAL ABSTRACTS, vol. 119, no. 9, 30. August 1993 (1993-08-30) Columbus, Ohio, US; abstract no. 95247y, Seite 1066; XP002122408 & C. H. LIN ET AL.: J. MED. CHEM., Bd. 36, Nr. 8, 1993, Seiten 1069-83, in der Anmeldung erwähnt

## Beschreibung

Die vorliegende Erfindung betrifft neue substituierte 2,3,3a,4,9,9a-Hexahydro-8-hydroxy-1H-benz[f]indol-Derivate, Verfahren zu ihrer Herstellung sowie ihre Verwendung als Arzneimittel. Die erfindungsgemäßen Indolderivate entsprechen der allgemeinen Formel **1**: 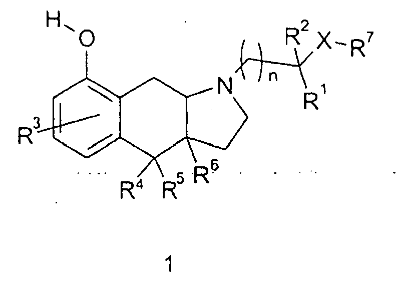 worin
- X: -O-, C₁-C₃-Alkylenoxy, -O-CH₂-CH₂-O- oder -O-CH₂-CH₂-NH-;
- R¹: Wasserstoff, Methyl, Ethyl oder Phenyl;
- R²: Wasserstoff oder Methyl;
- R³: Wasserstoff, F, Cl, Br, Hydroxy oder Methoxy;
- R⁴: Wasserstoff, Methyl oder Ethyl;
- R⁵: Wasserstoff, Methyl oder Ethyl;
- R⁶: Wasserstoff, Methyl oder Ethyl;
- R⁷: tert.-Butyl, Cylohexyl, 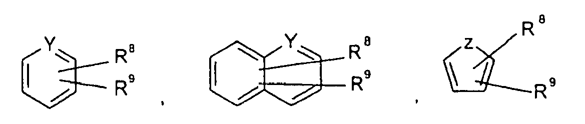
- n: eine ganze Zahl 0 oder 1;
- Y: N oder CH;
- Z: O, NH oder S;
- R⁸: Wasserstoff, Methyl, F, Cl, Br oder Methoxy;
- R⁹: Wasserstoff, Methyl, F, Cl, Br oder Methoxy
bedeuten können.

Gegenstand der Erfindung sind die einzelnen Stereoisomeren, deren Mischungen sowie die entsprechenden physiologisch geeigneten Säureadditionssalze mit anorganischen oder organischen Säuren - wie z.B. HCl oder HBr.

Beansprucht werden die enantiomerenreinen Verbindungen sowie die jeweiligen Racemate. Bevorzugt sind die jeweiligen trans-Derivate.

Die beschriebenen Verbindungen sind neu. Aus dem Stand der Technik sind 1-(C₁-C₈)-Alkyl, (C₁-C₈)-Alkenyl- bzw. (C₁-C₈)-Alkinyl-2,3,3a,4,9,9a-Hexahydro-1H-benz[f]indole bekannt [WO-91/00856, C.-H. Lin et. al. J. Med. Chem. 1993, 36, 1069-1083]. Diese Verbindungen wurden als D₂ und 5-HT_{1A} Agonisten charakterisiert und sind zur Behandlung einer Reihe von psychischen Erkrankungen sowie Stoffwechselerkankungen bekannt. Demgegenüber sind die hier beanspruchten Aralkyl-, Aryloxyalkyl-, Alkoxy-, Cycloalkylalkyl- und t-Butylalkyl-Verbindungen im Stand der Technik noch nicht erwähnt.

### Biologische Eigenschaften

Aus dem Stand der Technik ist bekannt, daß infolge von Hypoglykämie, Hypoxie, Anoxie, Trauma und lschämie auftretende Zellschäden und Funktionsausfälle z. T. auf einer erhöhten synaptischen Aktivität beruhen. Durch eine Reihe von Experimenten konnte gezeigt werden, daß solche hypoglykämischen und hypoxischen Zustände zu einer massiven Depolarisation der betroffenen Zellen führen. Durch diese Depolarisation wird wiederum ein pathogener Anstieg von intrazellulärem Kalcium und in neuronalen Gewebe zusätzlich eine erhöhte Freisetzung von exitatorischen Aminosäuren bewirkt. Der spannungsabhängige Natriumkanal hat eine Schlüsselrolle in dieser Kaskade. So kann durch dessen Blockade die Depolarisation der Zellen verhindert werden, wodurch der Kalciumeinstrom durch spannungsabhängige Kalciumkanäle und in neuronalen Gewebe zusätzlich auch durch NMDA-Rezeptorkanäle reduziert wird. Weiterhin wird durch den reduzierten Einstrom von Natriumionen in die Zelle verhindert, daß der Kalcium/Natrium-Austauscher gegenläufig arbeitet und Kalcium in die Zelle hineinfördert. Der reduzierte Einstrom von Natriumionen in die Zelle verhindert außerdem noch, daß der Glutamat-Transporter gegenläufig arbeitet und Glutamat freisetzt [C.P. Taylor und B.S. Meldrum, Trends Pharmacol. Sci., 16, 309-316 (1995); J. Urenjak und T.P. Obrenovitch Am. Soc. Pharmacol. Rev. 48, 21-67 (1996)].

Die beanspruchten Verbindungen sind Blocker des spannungsabhängigen Natriumkanals. Es handelt sich dabei um Verbindungen, die Batrachotoxin (BTX) mit hoher Affinitat besitzen (Kᵢ < 1000 nM) kompetitiv oder nicht-kompetitiv von der Bindungsstelle am Natriumkanal verdrängen. Solche Substanzen zeigen eine "use-dependency" bei der Blockade der Natriumkanäle, d.h. für die Bindung der Substanzen an dem Natriumkanal müssen die Natriumkanäle zunächst aktiviert werden. Die maximale Blockade der Natriumkanäle wird erst nach wiederholter Stimulation der Natriumkanäle erreicht. Demzufolge binden die Substanzen bevorzugt an Natriumkanäle, die vermehrt aktiviert werden. Dadurch sind die Substanzen in der Lage, bevorzugt in den Körperregionen wirksam zu werden, die pathologisch überstimuliert sind.

Als Testsystem für den Nachweis der Natriumkanal blockierenden Wirkung dient die BTX-Bindung am Natriumkanal [S.W. Postma & W.A. Catterall, Mol. Pharmacol 25, 219-227 (1984)] sowie patch-clamp Experimente, in denen gezeigt werden kann, daß die erfindungsgemäßen Verbindungen den elektrisch stimulierten Natriumkanal in einer "usedependent" Art und Weise blockieren [W.A. Catterall, Trends Pharmacol. Sci., 8, 57-65 (1987)].

Ferner wurde eine neuroprotektive Wirkung der erfindungsgemäßen Verbindungen durch die Blockade des Veratridin induzierten Glutamat-releases nachgewiesen [S. Villauneva, P. Frenz, Y. Dragnic, F. Orrego, Brain Res. 461, 377-380 (1988)]. Veratridin ist ein Toxin, das den Natriumkanal permanent öffnet. Dadurch kommt es, zu einem erhöhten Einstrom von Natriumionen in die Zelle. Über die oben beschriebene Kaskade führt dieser Natriumeinstrom in neuronalem Gewebe zu einer gesteigerten Freisetzung von Glutamat. Durch die erfindungsgemäßen Verbindungen läßt sich diese Glutamatfreisetzung antagonisieren.

Antikonvulsive Eigenschaften der erfindungsgemäßen Substanzen wurde durch eine protektive Wirkung gegen Krämpfe, die durch einen maximalen Elektoschock in Mäusen ausgelöst wurden, belegt [M. A. Rogawski & R.J. Porter, Pharmacol. Rev. 42, 223-286 (1990)], neuroprotektive Eigenschaften wurden durch eine protektive Wirkung in einem Ratten-MCAO-Modell belegt [U. Pschorn & A. J. Carter, J. Stroke Cerebrovascular Diseases, 6, 93-99 (1996)].

Ferner wurde beschrieben, daß Natriumkanalblocker zur Therapie der Zyklophrenie (manisch depressive Erkrankung) eingesetzt werden können [J. R. Calabrese, C. Bowden, M.J. Woyshville; in: Psychopharmacology: The Fourth Generation of Progress (Eds.: D. E. Bloom and D. J. Kupfer) 1099-1111. New York: Raven Press Ltd.]

Somit wurde belegt, daß Verbindungen der allgemeinen Formel **1** bei Erkrankungen eingesetzt werden können, deren Ursache auf eine durch Übererregung bedingte Funktionsstörung beruhen. Darunter fallen Erkrankungen wie Arrhythmien, Spasmen, kardiale und Gehirnischämien sowie neurodegenerative Erkrankungen verschiedener Genese; hierunter fallen beispielsweise: Status epileptikus, Hypoglykämie, Hypoxie, Anoxie, Gehirntrauma, Gehirnoedem, Gehirn-Schlaganfall, perinatale Asphyxie, amylotrophe laterale Sklerose, Huntington's Disease, Morbus Alzheimer, Morbus Parkinson, Zyklophrenie, Hypotonie, Herzinfakt, Herzrhythmusstörungen, Angina pectoris, Schmerzen verschiedener Formen bzw. Ätiologie - wie z.B.: Nozizeptorenschmerz mit Erregung von Schmerzrezeptoren u. Weiterleitung der Impulse an das ZNS oder. neuropathischer Schmerz. Infolge von Schädigungen des peripheren od. zentralen Nervensystems (z.·B. nach Amputation, Querschnittslähmung, Zoster oder bei diabetischer Polyneuropathie) Schmerzen infolge von funktionalen Störungen - wie z.·B. Migräne durch vaskuläre Fehlregulation, Rückenschmerzen durch körperliche Fehlhaltungen - einschließlich psychosomatischer Vorgänge wie z.B. Sympathikusaktivierung bei Angst, Muskelverspannung bei emotionalem Streß. In diesem Zusammenhang ist insbesondere ein Einsatz als Lokalanaesthetikum denkbar.

### Formulierungsbeispiele

### 1. Tabletten

| Zusammensetzung: | |
|---|---|
| Wirkstoff gemäß der Erfindung | 20 Gew.-Teile |
| Stearinsäure | 6 Gew.-Teile |
| Traubenzucker | 474 Gew.-Teile |

Die Bestandteile werden in üblicher Weise zu Tabletten von 500 mg Gewicht verarbeitet. Gewünschtenfalls kann der Wirkstoffgehalt erhöht oder vermindert und die Traubenzuckermenge entsprechend vermindert oder erhöht werden.

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 4. Infusionslösungen

Der erfindungsgemäße Wirkstoff wird in einer pharmazeutisch wirksamen Menge z.B. in einer aus dem Stand der Technik bekannten zur Infusion geeigneten physiologisch verträglichen Ausgangslösung - z.B. 5 %-ige Xylit- oder Mannit-Lösung - nach an sich aus dem Stand der Technik kekannten Verfahren eingebracht.

### 5. Inhalationspulver

Mikronisiertes Wirkstoffpulver (Verbindung der Formel I; Teilchengröße ca. 0,5 bis 7 µ m) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß DE-A 33 45 722, auf die hiermit inhaltlich Bezug genommen wird, inhaliert.

### Herstellungsverfahren

Schlüsselverbindungen bei der Synthese sind die Nor-Hexahydro-1H-benz[f]indole **2a,** das hier als das entsprechende (-)-Enantiomere dargestellt ist, sowie **3a** und **3b**.

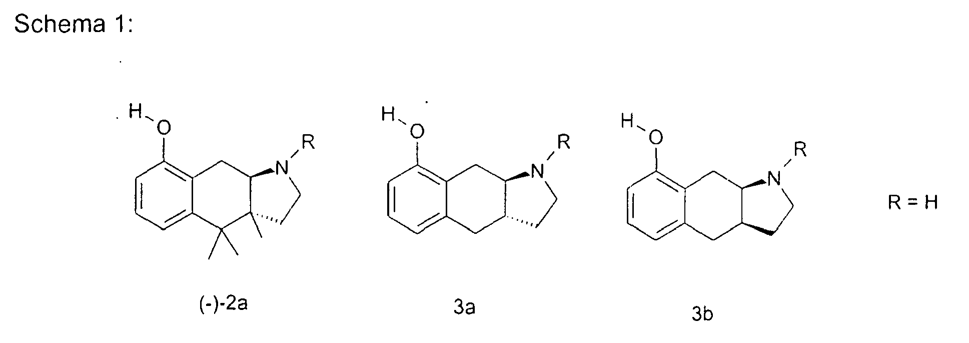

Verbindung **2a** wurde nach dem in Schema 2 beschriebenen Verfahren erhalten.

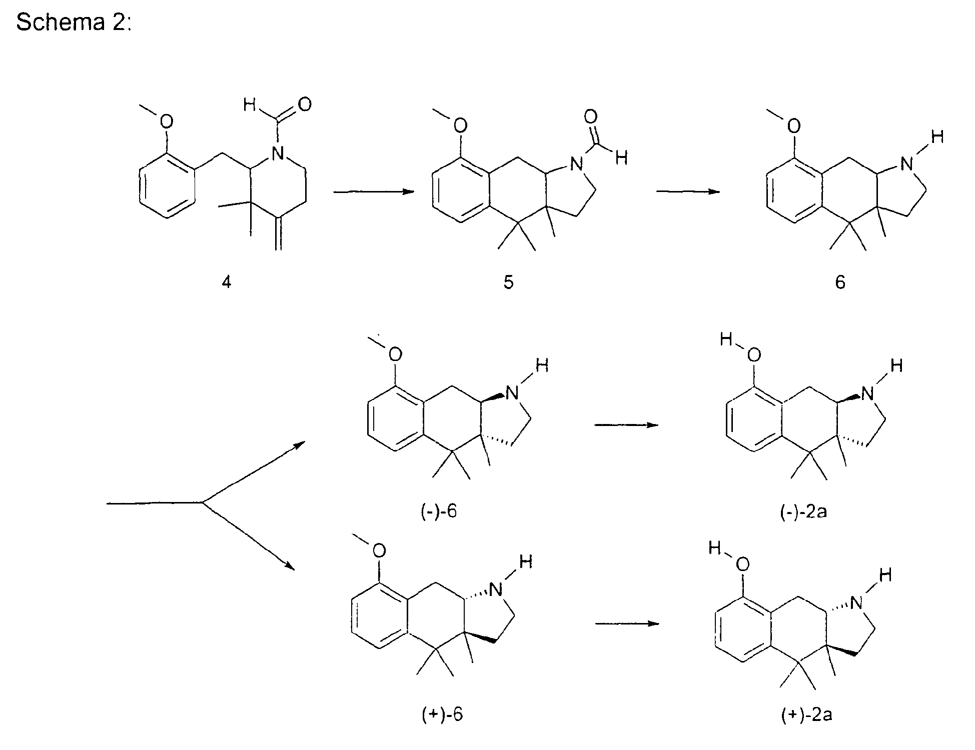

Die Synthese von **3a** und **3b** kann analog dem in WO-A-91/00856 beschriebenen Verfahren erfolgen. Die Synthese ist in Schema 3 dargestellt.

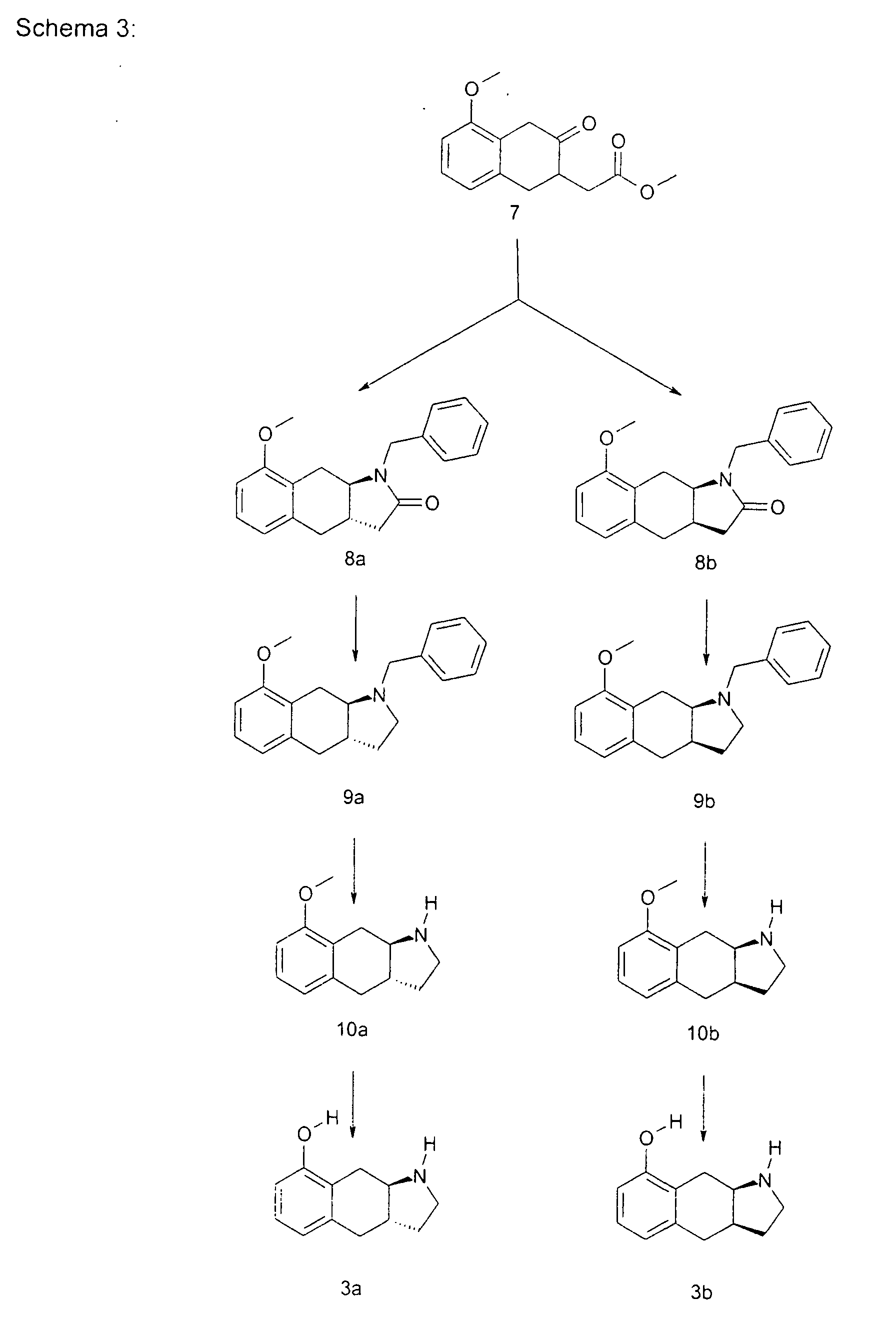

Die Einführung des N-Substituenten erfolgt durch Umsetzung der Schlüsselverbindungen **2a, 3a** und **3b** mit Acylierungsmitteln zu den Zwischenverbindungen **11** und deren anschließender Reduktion oder durch direkte Alkylierung der Schlüsselverbindungen **2a** bzw. **3a** oder **3b** mit Alkylierungsmitteln oder durch Umsetzung mit Aldehyden zu **12** und anschließender Reduktion. In Schema 4 sind diese Wege beispielhaft für die Schlüsselverbindung (-)-**2a** angegeben.

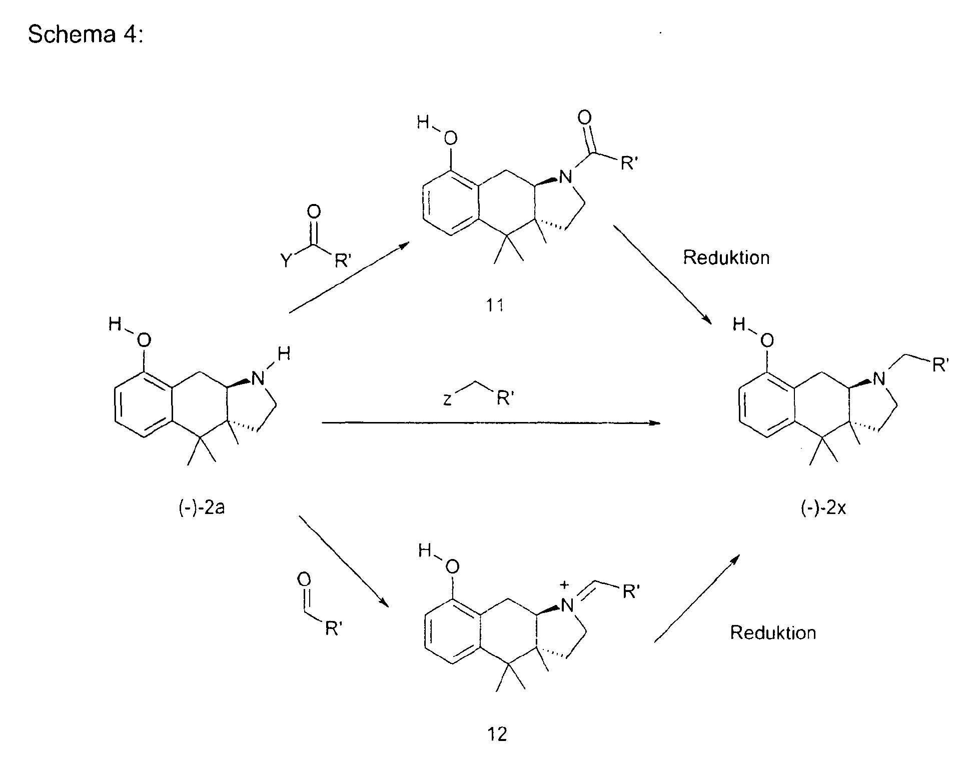

Die erfindungsgemäßen Verbindungen können anschließend regioselektiv in der Benzindol-Partialstruktur substituiert werden. Ein Beispiel ist für Verbindung (-)-**2b** in Schema 5 angegeben.

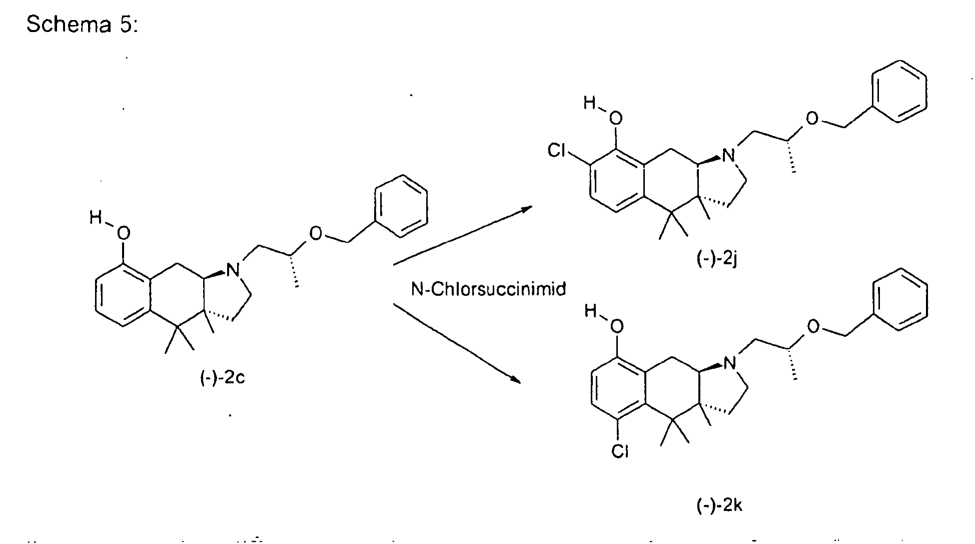

Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

### Beispiele:

Bei den mit * gekennzeichneten Verbindungen handelt es sich um zwischenprodukte bzw. Vergleichsbeispiele.

### Beispiel 1:

### trans-1-Formyl-8-methoxy-3a,4,4-trimethyl-2,3,3a,4,9,9a-hexahydro-1H-benz[f]indol (5)*

Zu einer Suspension von 88,0 g (0,66 mol) Aluminiumtrichlorid in 420 mL absolutem Dichlormethan wird bei 0-5 °C eine Lösung von 60,1 g (0,22 mol) N-Formyl-2-(2'-methoxyphenyl)methyl-3,3-dimethyl-4-methylen-piperidin (**4**) in 200 mL Dichlormethan innerhalb von 2 h zugetropft. Man läßt noch 3 h bei 5 °C nachreagieren und gibt den Ansatz auf 800 mL Eis. Die organische Phase wird abgetrennt und die wäßrige Phase noch zweimal mit je 250 mL Dichlormethan extrahiert. Die vereinigten organischen Phasen werden über MgSO₄ getrocknet und das Lösungsmittel im Vakuum abgezogen. Der Rückstand wird an Kieselgel chromatographiert (500 g Kieselgel, Lösungsmittel: Essigester). Man erhält 36,0 g (60%) trans-1-Formyl-8-methoxy-3a,4,4-trimethyl-2,3,3a,4,9,9a-hexahydro-1H-benz[f]indol (**5**) als Öl.

### Beispiel 2:

### trans-8-Methoxy-3a,4,4-trimethyl-2,3,3a,4,9,9a-hexahydro-1H-benz[f]indol (6)*

9,6 g (35 mmol) trans-1-Formyl-8-methoxy-3a,4,4-trimethyl-2,3,3a,4,9,9a-hexahydro-1H-benz[f]indol (**5**) werden in 75 mL n-Propanol gelöst und mit 25 mL konz. Salzsäure und 15 mL Wasser versetzt. Man erhitzt 12 h unter Rückfluß, zieht den Alkohol im Vakuum ab und verdünnt mit 800 mL Wasser. Man extrahiert einmal mit 150 mL Essigester (verwerfen), stellt die wäßrige Phase mit Natriumhydroxyd alkalisch und extrahiert dreimal mit je 200 mL Essigester. Die vereinigten organischen Phasen werden über MgSO₄ getrocknet und das Lösungsmittel im Vakuum abgezogen. Man erhält 8,6 g (100%) trans-8-Methoxy-3a,4,4-trimethyl-2,3,3a,4,9,9a-hexahydro-1H-benz[f]indol (**6**) als Öl.

### Beispiel 3:

### (-)-1 R- trans-8-Methoxy-3a,4,4-trimethyl-2,3,3a,4,9,9a-hexahydro-1H-benz[f]indol-Malat ((-)6MA)* und (+)-1 S-trans-Methoxy-3a,4,4-trimethyl-2,3,3a,4,9,9a-hexahydro-1H-benz[f]indolmalat ((+)6MA)*

8,6 g (35 mmol) trans-8-Methoxy-3a,4,4-trimethyl-2,3,3a,4,9,9a-hexahydro-1H-benz[f]indol (**6**) werden in 89 mL Methanol gelöst und mit einer Lösung von 8,5 g (63 mmol) L-(-)-Äpfelsäure in 85 mL Methanol versetzt. Man läßt 1 h bei RT rühren, saugt die ausgefallenen Kristalle ab und kristallisiert aus 200 mL Methanol um. Man erhält 4,8 g (35%) (-)-1 R-trans-8-Methoxy-3a,4,4-trimethyl-2,3,3a,4,9,9a-hexahydro-1H-benz[f]indol-malat ((-)**6**MA), Schmp.: 214 °C, [α]_{D}²⁵ = -42,5° (c = 1, Methanol).

Die Mutterlauge wird eingeengt, mit konz. Ammoniak alkalisch gestellt und zweimal mit je 200 mL Essigester extrahiert. Die vereinigten organischen Phasen werden über MgSO₄ getrocknet und das Lösungsmittel im Vakuum abgezogen. Anschließend wird unter Verwendung von D-(+)-Äpfelsäure (+)-1 S-trans-Methoxy-3a,4,4-trimethyl-2,3,3a,4,9,9a-hexahydro-1H-benz[f]indol-malat ((+)**6**MA) isoliert. Ausbeute: 3,5 g (25%), Schmp.: 214 °C, [α]_{D}²⁵ = +46,1° (c = 1, Methanol).

### Beispiel 4:

### (-)-1R-trans-8-Hydroxy-3a,4,4-trimethyl-2,3,3a,4,9,9a-hexahydro-1H-benz[f]indol-hydrobromid ((-)2aBr)*

4,8 g (12,8 mmol) (-)-1 R-trans-8-Methoxy-3a,4,4-trimethyl-2,3,3a,4,9,9a-hexahydro-1H-benz[f]indol-malat ((-)**6**MA) werden zunächst mit konz. Ammoniak in die freie Base überführt. Anschließend wird mit 8 mL Wasser und 25 mL 62%ige Bromwasserstoffsäure versetzt und 3 h unter Rückfluß erhitzt. Anschließend wird im Vakuum eingeengt und der Rückstand aus 30 mL Aceton umkristallisiert. Ausbeute: 3,9 g (98%), Schmp.: > 250 °C, [α]_{D}²⁵ = - 68,4° (c = 1, Methanol).

Analog zu Beispiel 4 wird hergestellt:
(+)-1 R-trans-8-Hydroxy-3a,4,4-trimethyl-2,3,3a,4,9,9a-hexahydro-1H-benz[f]indol-hydrobromid ((+)**2a**Br)*

Es werden 19,3 g (51 mmol) (+)-1R-trans-8-Methoxy-3a,4,4-trimethyl-2,3,3a,4,9,9a-hexahydro-1H-benz[f]indol-malat ((+)**6**MA) eingesetzt. Ausbeute: 14,4 (91 %), Schmp.: > 250 °C, [α]_{D}²⁵ = + 70,0° (c = 1, Methanol).

### Beispiel 5:

### trans-(±)-1-Benzyl-8-methoxy-2,3,3a,4,9,9a-hexahydro-1H-benz[f]indol-2-on ((8a) und cis-(±)-1-Benzyl-8-methoxy-2,3,3a,4,9,9a-hexahydro-1H-benz[f]indol-2-on (8b)*

3,6 g (14,5 mmol) (±)-1,2,3,4-Tetrahydro-5-methoxy-3-oxo-2-naphthalin-essigsäuremethylester (**7**) und 7,8 g (73 mmol) Benzylamin werden in 50 mL THF und 50 mL Methanol gelöst. Man kühlt auf 0 °C, säuert mit Eisessig auf pH 4,5 an und läßt 30 min. rühren. Anschließend wurde mit 1,8 g (29 mmol) Natriumcyanoborhydrid versetzt und 3 d bei Raumtemperatur gerührt. Die Reaktionsmischung wird mit 20%iger Natronlauge gequenched und das organische Lösungsmittel im Vakuum abgezogen. Anschließend wird dreimal mit je 150 mL Dichlormethan extrahiert, die vereinigte organische Phase einmal mit gesättigter Natriumchloridlösung gewaschen, über MgSO₄ getrocknet und das Lösungsmittel im Vakuum abgezogen. Der Rückstand wird an Kieselgel chromatographiert (ca. 1000 g Kieselgel, Essigester/Cyclohexan 1:1). Man erhält 0,5 g (10%) **8a** und 1,1 g (25%) **8b** als Öl.

### Beispiel 6:

### cis-(±)-1-Benzyl-8-methoxy-2,3,3a,4,9,9a-hexahydro-1H-benz[f]indol ((9b)*

0,51 g (13,4 mmol) LiAlH₄ werden in 5 mL THF vorgelegt, auf 0 °C gekühlt und eine Lösung von 1,0 g (3,3 mmol) cis-(±)-1-Benzyl-8-methoxy-2,3,3a,4,9,9a-hexahydro-1H-benz[f]indol-2-on ((**8b**) in 20 mL THF zugetropft. Anschließend wird 5 h unter Rückfluß erhitzt, auf Raumtemperatur abgekühlt und mit 100 mL gesättigter Natriumsulfatlösung versetzt. Man extrahiert dreimal mit je 100 mL Essigester, trocknet die vereinigte organische Phase über MgSO₄ und zieht das Lösungsmittel im Vakuum ab. Man suspendiert den Rückstand in 15 mL Methanol, saugt von nicht löslichen Bestandteilen ab und zieht das Lösungsmittel im Vakuum ab. Man erhält 0,5 g (52%) **9b** als Öl.

Analog zu Beispiel 6 wird hergestellt:
trans-(±)-1-Benzyl-8-methoxy-2,3,3a,4,9,9a-hexahydro-1H-benz[f]indol (**9a**)*

Es werden 0,21 g (5,5 mmol) LiAlH₄ und 0,5 g (1,6 mmol) trans-(±)-1-Benzyl-8-methoxy-2,3,3a,4,9,9a-hexahydro-1 H-benz[f]indol-2-on (**8a**) eingesetzt. Ausbeute: 0,4 g (85%).

### Beispiel 7:

### cis-(±)-8-Methoxy-2,3,3a,4,9,9a-hexahydro-1H-benz[f]indol (10b)*

1,7 g (5,8 mmol) cis-(±)-1-Benzyl-8-methoxy-2,3,3a,4,9,9a-hexahydro-1H-benz[f]indol (**9b**) werden in 35 mL Methanol gelöst und bei 20 °C und 5 bar Wasserstoffdruck an 0,2 g Pd/C (10%) hydriert. Nach 5 h filtriert man über Kieselgel und zieht das Lösungsmittel im Vakuum ab. Man erhält 1,2 g (100%) **10b** als Öl.

Analog zu Beispiel 7 wird hergestellt:
trans-(±)-8-Methoxy-2,3,3a,4,9,9a-hexahydro-1H-benz[f]indol (**10a**)*

Es werden 0,47 g (1,6 mmol) trans-(±)-1-Benzyl-8-methoxy-2,3,3a,4,9,9a-hexahydro-1H-benz[f]indol (**9a**) eingesetzt. Man erhält 0,32 g (100%) **10a** als Öl.

### Beispiel 8:

### cis-(±)-8-Hydroxy-2,3,3a,4,9,9a-hexahydro-1H-benz[f]indol-hydrobromid (3bBr)*

0,55 g (2,7 mmol) cis-(±)-8-Methoxy-2,3,3a,4,9,9a-hexahydro-1H-benz[f]indol (**10b**) werden in 6 mL Wasser und 12 mL konz. Bromwasserstoffsäure gegeben und 20 h unter Rückfluß erhitzt.. Anschließend wird im Vakuum eingeengt, der Rückstand nochmals in 10 mL Ethanol aufgenommen und das Lösungsmittel erneut im Vakuum abgezogen. Man erhält 0,69 g (95%) des gewünschten Produkts als Hydrobromid. Analog zu Beispiel 8 wird hergestellt:
trans-(±)-8-Hydroxy-2,3,3a,4,9,9a-hexahydro-1H-benz[f]indol-hydrobromid (**3a**Br)*

Es werden 0,32 g (1,6 mmol) trans-(±)-8-Methoxy-2,3,3a,4,9,9a-hexahydro-1H-benz[f]indol (**10a**) eingesetzt. Ausbeute: 0,41 g (95%).

### Beispiel 9:

### (-)-(1 R,2'R)-trans-1-(2'-Benzyloxy)propyl-8-hydroxy-3a,4,4-trimethyl-2,3,3a,4,9,9a-hexahydro-1H-benz[f]indol-hydrochlorid ((-)2bHCl)

4,65 g (15 mmol) (-)-1 R-trans-Hydroxy-3a,4,4-trimethyl-2,3,3a,4,9,9a-hexahydro-1H-benz[f]indol-hydrobromid ((-)**2a**HBr) und 5,0 g (39 mmol) Triethylamin werden in 35 mL Dichlormethan gelöst und nach 30 min. eine Lösung von 3,6 g (18 mmol) (+)-R-2-Benzyloxypropionsäurechlorid in 10 mL Dichlormethan zugetropft. Man läßt noch 2 h bei Raumtemperatur rühren, versetzt mit 20 mL 2 N Salzsäure und trennt die organische Phase ab. Die organische Phase wird über MgSO₄ getrocknet, das Lösungsmittel im Vakuum abgezogen und der Rückstand in 60 mL Tetrahydrofuran aufgenommen. Zu dieser Lösung werden 1,9 g (50 mmol) LiAlH₄ hinzugegeben wobei die Temperatur auf 35 °C ansteigt. Man läßt 1 h nachreagieren, versetzt mit 20 mL 40%ige Ammoniumtartratlösung, trennt die organische Phase ab und extrahiert zweimal mit je 100 mL Essigester. Die vereinigten organischen Phasen werden über MgSO₄ getrocknet und das Lösungsmittel im Vakuum abgezogen. Der Rückstand wird in 50 mL Aceton aufgenommen und mit etherischer Salzsäure das Hydrochlorid gefällt. Die Kristalle werden abgetrennt und mit Aceton gewaschen. Ausbeute: 2.8 g (45%), Schmp.: 236 °C, [α]_{D}²⁵ = (-) 124,3° (C=1 in Methanol).

Analog zu Beispiel 9 werden hergestellt:
(-)-(1 R,2'S)-trans-1-(2'-Benzyloxy)propyl-8-hydroxy-3a,4,4-trimethyl-2,3,3a,4,9,9a-hexahydro-1 H-benz[f]indol-hydrochlorid ((-)**2c**Cl)
(-)-(1 R,2'S)-trans-1-(2'-Benzyloxy)propyl-8-hydroxy-3a,4,4-trimethyl-2,3,3a,4,9,9a-hexahydro-1 H-benz[f]indol-hydrochlorid ((-)**2c**Cl)
   Es werden 5,9 g (19 mmol) (-)-1 R -trans-Hydroxy-3a,4,4-trimethyl-2,3,3a,4,9,9a-hexahydro-1H-benz[f]indol-hydrobromid ((-)**2a**HBr) und 3,8 g (19 mmol) (-)-S-2-Benzyloxypropionsäurechlorid eingesetzt. Ausbeute: 3,2 g (40%), Schmp.: > 250 °C, [α]_{D}²⁵ = (-) 11,9° (C=1 in Methanol).
(+)-(1S,2'S)-trans-1-(2'-Benzyloxy)propyl-8-hydroxy-3a,4,4-trimethyl-2,3,3a,4,9,9a-hexahydro-1H-benz[f]indol-hydrochlorid ((+)**2b**HCl)
   Es werden 1,6 g (5 mmol) (+)-1 R-trans-Hydroxy-3a,4,4-trimethyl-2,3,3a,4,9,9a-hexahydro-1H-benz[f]indol-hydrobromid ((+)**2a**Br) und 1,0 g (5,0 mmol) (-)-S-2-Benzyloxypropionsäurechlorid eingesetzt. Ausbeute: 1,3 g (63%), Schmp.: 236 °C, [α]_{D}²⁵ = (+) 124,8° (C=1 in Methanol).
(+)-(1S,2'R)-trans-1-(2'-Benzyloxy)propyl-8-hydroxy-3a,4,4-trimethyl-2,3,3a,4,9,9a-hexahydro-1H-benz[f]indol-hydrochlorid ((+)**2c**HCl)
   Es werden 1,6 g (5 mmol) (+)-1 R-trans-Hydroxy-3a,4,4-trimethyl-2,3,3a,4,9,9a-hexahydro-1H-benz[f]indol-hydrobromid ((+)**2a**Br) und 1,0 g (5 mmol) (+)-R-2-Benzyloxypropionsäurechlorid eingesetzt. Ausbeute: 1,0 g (48%), Schmp.: > 250 °C, [α]_{D}²⁵ = (+) 12,7° (C=1 in Methanol).
(+)-(1S,2'S)-trans-8-Hydroxy -1-(2'-methoxy-2"-phenyl)ethyl -3a,4,4-trimethyl-2,3,3a,4 ,9,9a-hexahydro-1H-benz[f]indol-hydrochlorid ((+)**2d**HCl)*
   Es werden 1,6 g (5 mmol) (+)-1R-trans-Hydroxy-3a,4,4-trimethyl-2,3,3a,4,9,9a-hexahydro-1H-benz[f]indol-hydrobromid ((+)**2a**Br) und 0,9 g (5 mmol) (+)-S-2-Methoxyphenylessigsäurechlorid eingesetzt. Ausbeute: 0,9 g (45%), Schmp.: > 250 °C, [α]_{D}²⁵ = (+) 174,2° (C=1 in Methanol)..
(-)-(1 R)-trans-8-Hydroxy-1-(2'-phenoxy)ethyl-3a,4,4-trimethyl-2,3,3a,4,9,9a-hexahydro-1 H-benz[f]indol-hydrochlorid ((-)**2e**HCl)
   Es werden 1,6 g (5 mmol) (-)-1 R-trans-Hydroxy-3a,4,4-trimethyl-2,3,3a,4,9,9a-hexahydro-1H-benz[f]indol-hydrobromid ((-)**2a**HBr) und 0,8 g (6 mmol) 2-Phenoxyessigsäurechlorid eingesetzt. Ausbeute: 0,9 g (46%), Schmp.: 248 °C, [α]_{D}²⁵ = (-) 69,3° (C=1 in Methanol).
(+)-(1 S)-trans-8-Hydroxy-1-(2'-phenoxy)ethyl-3a,4,4-trimethyl-2,3,3a,4,9,9a-hexahydro-1H-benz[f]indol-hydrochlorid ((+)**2e**HCl)
   Es werden 1,6 g (5 mmol) (+)-1 S-trans-Hydroxy-3a,4,4-trimethyl-2,3,3a,4,9,9a-hexahydro-1 H-benz[f]indol-hydrobromid ((+)**2a**HBr) und 0,8 g (6 mmol) 2-Phenoxyessigsäurechlorid eingesetzt. Ausbeute: 1,2 g (62%), Schmp.: 254 °C, [α]_{D}²⁵ = (+) 71,5° (C=1 in Methanol).
trans-(±)-8-Hydroxy-1-(2'-(2"-phenoxy)ethoxy)ethyl-2,3,3a,4,9,9a-hexahydro-1H-benz[f]indol-oxalat (**3e**OX)
   Es werden 0,2 g (0,74 mmol) trans-(±)-8-Hydroxy-2,3,3a,4,9,9a-hexahydro-1H-benz[f]indol-hydrobromid (**3a**Br) und 0,2 g (0,93 mmol) 2-(2'-Phenoxy)ethoxy)essigsäurechlorid eingesetzt. Die Base wird mit etherischer Oxalsäure in das Oxalat überführt. Ausbeute: 0,16 g (55%), Schmp.: 181 °C.
cis-(±)-8-Hydroxy-1-(2'-(2"-phenoxy)ethoxy)ethyl-2,3,3a,4,9,9a-hexahydro-1H-benz[f]indol-oxalat (**3f**OX)
   Es werden 0,63 g (2,3 mmol) cis-(±)-8-Hydroxy-2,3,3a,4,9,9a-hexahydro-1H-benz[f]indol-hydrobromid (**3b**HBr) und 0,5 g (2,6 mmol) 2-(2'-Phenoxy)ethoxy)essigsäurechlorid eingesetzt. Die Base wird mit etherischer Oxalsäure in das Oxalat überführt. Ausbeute: 0,3 g (33%), Schmp.: 176 °C.

### Beispiel 10:

### (+)-(1S,2'S)-trans-1-(2'-(2",6"-Difluorbenzyloxy)propyl-8-hydroxy-3a,4,4-trimethyl-2,3,3a,4,9,9a-hexahydro-1H-benz[f]indol-hydrochlorid ((+)2fHCl)

1,46 g (4,7 mmol) (+)-1 R-trans-Hydroxy-3a,4,4-trimethyl-2,3,3a,4,9,9a-hexahydro-1H-benz[f]indol-hydrobromid ((+)**2a**HBr) und 1,0 g (4,6 mmol) (-)-S-2-(2',6'-Difluorbenzyloxy)propionsäure werden in einer Mischung aus 50 mL Tetrahydrofuran und 50 mL Dichlormethan gelöst und mit 0,8 g Benzotriazol und 4 mL Ethyldiisopropylamin versetzt. Dann werden 0,9 g TBTU hinzugefügt und 2 h bei RT gerührt. Anschließend zieht man das Lösungsmittel im Vakuum ab, nimmt in 100 mL Essigester auf und extrahiert je einmal mit 100 mL gesättigter Kaliumhydrogencarbonatlösung und 100 mL 1 N Salzsäurelösung. Die organische Phase wird über MgSO₄ getrocknet und das Lösungsmittel im Vakuum abgezogen. Der Rückstand wird in 20 mL THF aufgenommen und mit 0,5 g (13 mmol) LiAlH₄ versetzt. Man läßt 1 h bei 50 °C nachreagieren, versetzt mit 50 mL 40%ige Ammoniumtartratlösung, trennt die organische Phase ab und extrahiert zweimal mit je 100 mL Essigester. Die vereinigten organischen Phasen werden über MgSO₄ getrocknet und das Lösungsmittel im Vakuum abgezogen. Der Rückstand wird in 50 mL Aceton aufgenommen und mit etherischer Salzsäure das Hydrochlorid gefällt. Die Kristalle werden abgetrennt und mit Aceton gewaschen. Ausbeute: 1,3 g (63%), Schmp.: 242 °C, [α]_{D}²⁵ = (+) 1,16,4° (C=1 in Methanol).

Analog zu Beispiel 10 werden hergestellt:
(-)-(1 R,2'S)-trans-1-(2'-(2",6"-Difluorbenzyloxy)propyl-8-hydroxy-3a,4,4-trimethyl-2,3,3a,4,9,9a-hexahydro-1H-benz[f]indol-hydrochlorid ((+)**2g**Cl7HCl)
   Es werden 1,5 g (4,7 mmol) (-)-1 R-trans-Hydroxy-3a,4,4-trimethyl-2,3,3a,4,9,9a-hexahydro-1 H-benz[f]indol-hydrobromid ((-)**2a**Br) und 1,0 g (4,6 mmol) (-)-S-2-(2',6'-Difluorbenzyloxy)propionsäure eingesetzt. Ausbeute: 1,3 g (63%), Schmp.: 260 °C, [α]_{D}²⁵ = (-) 33,8° (C=1 in Methanol).
(1 RS,2'S)-trans-1-(2'-Benzyloxy)propyl-8-hydroxy-2,3,3a,4,9,9a-hexahydro-1H-benz[f]indol-oxalat (**3c**OX)
   Es werden 0,2 g (0,74 mmol) trans-(±)-8-Hydroxy-2,3,3a,4,9,9a-hexahydro-1H-benz[f]indol-hydrobromid (**3a**Br) und 0,13 g (0,74 mmol) (-)-S-(2-(Benzyloxy)propionsäure eingesetzt. Die Base wird mit etherischer Oxalsäure in das Oxalat überführt. Ausbeute: 0,13 g (46%), Schmp.: 193 °C.
(1RS,2'S)-cis-1-(2'-Benzyloxy)propyl-8-hydroxy-2,3,3a,4,9,9a-hexahydro-1H-benz[f]indol-oxalat (**3d**OX)
   Es werden 0,46 g (1,7 mmol) cis-(±)-8-Hydroxy-2,3,3a,4,9,9a-hexahydro-1H-benz[f]indol-hydrobromid ((**3b**HBr) und 0,31 g (1,7 mmol) (-)-S-(2-(Benzyloxy)propionsäure eingesetzt. Die Base wird mit etherischer Oxalsäure in das Oxalat überführt. Ausbeute: 0,25 g (38%).

### Beispiel 11:

### (-)-(1R)-trans-8-Hydroxy-1-(2'-(2"-phenoxy)ethoxy)ethyl-3a,4,4-trimethyl-2,3,3a,4,9,9a-hexahydro-1H-benz[f]indol-hydrochlorid ((-)2hHCl)

3,1 g (10 mmol) (-)-1 R-trans-Hydroxy-3a,4,4-trimethyl-2,3,3a,4,9,9a-hexahydro-1H-benz[f]indol-hydrobromid ((-)**2a**Br) und 2,2 g (11 mmol) 2-(2-Phenoxy)ethoxy-ethylchlorid werden in 50 mL (Dimethylformamid) DMF gelöst, eine katalytische Menge Kl und 3 g Natriumcarbonat hinzugefügt. Die Mischung wird 57h unter Rückfluß erhitzt. Anschließend wird von den anorganischen Salzen abgesaugt und das Lösungsmittel im Vakuum abgezogen. Der Rückstand wird in 100 mL Wasser aufgenommen, dreimal mit je 100 mL Essigester extrahiert und die vereinigten organischen Extrakte werden noch einmal mit 50 mL Wasser gewaschen, über MgSO₄ getrocknet und das Lösungsmittel im Vakuum abgezogen. Der Rückstand wird über eine kurze Kieselgelsäule filtriert (25 g Kieselgel, 300 mL Essigester) in Aceton gelöst und mit etherischer Salzsäure das Hydrochlorid gefällt. Ausbeute: 3,3 g (76%), Schmp.: 186 °C. [α]_{D}²⁵ = (-) 72.1° (c=1 in Methanol).

Analog zu Beispiel 11 werden dargestellt:
(+)-(1 S)-trans-8-Hydroxy-1-(2'-(2"-phenoxy)ethoxy)ethyl-3a,4,4-trimethyl-2,3,3a,4,9,9a-hexahydro-1H-benz[f]indol-hydrochlorid ((+)**2h**HCl)
   Es werden 3,1 g (10 mmol) (+)-1R-trans-Hydroxy-3a,4,4-trimethyl-2,3,3a,4,9,9a-hexahydro-1 H-benz[f]indol-hydrobromid ((+)**2a**HBr) und 2,2 g (11 mmol) 2-(2-Phenoxy)ethoxy-ethylchlorid eingesetzt. Ausbeute: 3,2 g (74%), Schmp.: 186 °C, [α]_{D}²⁵ = (+) 71,4° (c=1 in Methanol).
(+)-(1S)-trans-8-Hydroxy-1-(2'-(2"-(8'"-chinolinoxy)ethoxy)ethyl-3a,4,4-trimethyl-2,3,3a,4,9,9a-hexahydro-1H-benz[f]indol-hydrochlorid ((+)**2i**HCl)
   Es werden 1,25 g (4 mmol) (+)-1R-trans-Hydroxy-3a,4,4-trimethyl-2,3,3a,4,9,9a-hexahydro-1 H-benz[f]indol-hydrobromid ((+)**2a**HBr) und 1,2 g (4 mmol) 2-(2'-(8"-Chinolinoxy)ethoxy)ethylchlorid eingesetzt. Ausbeute: 0,8 g (40%), Schmp.: 167 °C, [α]_{D}²⁵ = (+) 44,2° (c=1 in Methanol).
(-)-(1R)-trans-8-Hydroxy-1-(2'-(2"-(8'"-Chinolinoxy)ethoxy)ethyl-3a,4,4-trimethyl-2,3,3a,4,9,9a-hexahydro-1 H-benz[f]indol ((-)**2i**)
   Es werden 1,25 g (4 mmol) (-)-1R-trans-Hydroxy-3a,4,4-trimethyl-2,3,3a,4,9,9a-hexahydro-1H-benz[f]indol-hydrobromid ((-)**2a**Br) und 1,2 g (4 mmol) 2-(2'-(8"'-Chinolinoxy)ethoxy)ethylchlorid eingesetzt. Die freie Base wird aus Essigester/ Cyclohexan umkristallisiert. Ausbeute: 0,7 g (39%), Schmp.: 163 °C, [α]_{D}²⁵ = (-) 84,8° (c=1 in Methanol).

### Beispiel 12:

### (+)-(1 S)-trans-1-(3-Furanyl)methyl-8-hydroxy-3a,4,4-trimethyl-2,3,3a,4,9,9a-hexahydro-1H-benz[f]indol-hydrochlorid ((+)2jHCl)*

0,93 g (4 mmol) (+)-1 R-trans-Hydroxy-3a,4,4-trimethyl-2,3,3a,4,9,9a-hexahydro-1H-benz[f]indol ((+)**2a**) und 0,6 mL (7,2 mmol) 3-Furanylaldehyd werden in 10 mL Methanol gelöst, mit Molekularsieb versetzt und 2 h bei RT gerührt. Anschließend wird vom Molekularsieb abfiltriert und das Filtrat mit 0,31 g (5 mmol) Natriumcyanoborhydrid und 1,2 mL Eisessig versetzt. Man läßt über Nacht stehen, versetzt mit 20 mL 4 N Salzsäure und engt im Vakuum ein. Der Rückstand wird in Aceton gelöst und mit etherischer Salzsäure das Hydrochlorid gefällt. Ausbeute: 0,7 g (50%), Schmp.: > 250 °C, [α]_{D}²⁵ = (+) 99,8° (C=1 in Methanol).

Analog zu Beispiel 12 wird hergestellt:
(-)-(1R)-trans-1-(3-Furanyl)methyl-8-hydroxy-3a,4,4-trimethyl-2,3,3a,4,9,9a-hexahydro-1H-benz[f]indol-hydrochlorid ((-)**2j**HCl)*
   Es werden 0,93 g (4 mmol) (-)-1 R-trans-Hydroxy-3a,4,4-trimethyl-2,3,3a,4,9,9a-hexahydro-1H-benz[f]indol ((-)**2a**) und 0,6 mL (7,2 mmol) 3-Furanylaldehyd eingesetzt. Ausbeute: 0,7 g (50%), Schmp.: > 250 °C, [α]_{D}²⁵ = (-) 102,3° (C=1 in Methanol).

### Beispiel 13:

### (-)-(1R,2'S)-trans-1-(2'-Benzyloxy)propyl-7-chlor-8-hydroxy-3a,4,4-trimethyl-2,3,3a,4,9,9a-hexahydro-1H-benz[f]indol-hydrochlorid ((-)2jHCl) und (-)-(1 R,2'S)-trans-1-(2'-Benzyloxy)propyl-5-chlor-8-hydroxy-3a,4,4-trimethyl-2,3,3a,4,9,9a-hexahydro-1 H-benz[f]indol-hydrochlorid ((-)2kHCl)

1,0 g (2,4 mmol) (-)-(1R,2'S)-trans-1-(2'-Benzyloxy)propyl-8-hydroxy-3a,4,4-trimethyl-2,3,3a,4,9,9a-hexahydro-1H-benz[f]indol-hydrochlorid ((-)**2c**HCl) und 0,3 g (2,5 mmol) N-Chlorsuccinimid werden in 20 mL Eisessig suspendiert und 24 h bei RT gerührt wobei die Suspension in Lösung geht. Anschließend wird der Ansatz im Vakuum eingeengt, der Rückstand mit 100 mL eiskalter 2 N Natronlauge versetzt und dreimal mit je 100 mL Essigester ausgeschüttelt. Die vereinigten organischen Phasen werden über MgSO₄ getrocknet und das Lösungsmittel im Vakuum abgezogen. Anschließend wird der Rückstand an Aluminiumoxid chromatographiert (Dichlormethan/ Methanol 19: 1). Die geeigneten Fraktionen werden eingeengt und der Rückstand in 15 mL Aceton gelöst und mit etherischer Salzsäure das Hydrochlorid gefällt. Man erhält 0.4 g (37%) (-)-**2j**HCl, Schmp.: 218 °C, []_{D}²⁵ = (-) 12,3° (C=1 in Methanol) und 0.44 g (41%) (-)-**2k**HCl, Schmp.: > 250 °C, []_{D}²⁵ = (-) 14,4° (C=1 in Methanol).

Analog zu Beispiel 13 wird hergestellt:
(-)-(1 R,2'R)-trans-1-(2'-Benzyloxy)propyl-7-chlor-8-hydroxy-3a,4,4-trimethyl-2,3,3a,4,9,9a-hexahydro-1H-benz[f]indol-hydrochlorid ((-)**2l**HCl) und (-)-(1 R,2'R)-trans-1-(2'-Benzyloxy)propyl-5-chlor-8-hydroxy-3a,4,4-trimethyl-2,3,3a,4,9,9a-hexahydro-1 H-benz[f]indol-hydrochlorid ((-)**2m**HCl)
   Es werden 0,42 g (1,0 mmol) (-)-(1 R,2'S)-trans-1-(2'-Benzyloxy)propyl-8-hydroxy-3a,4,4-trimethyl-2,3,3a,4,9,9a-hexahydro-1H-benz[f]indol-hydrochlorid ((-)**2c**Cl) und 0,13 g (1,1 mmol) N-Chlorsuccinimid eingesetzt. Man erhält 0.1 g (22%) (-)-**2l**HCl, Schmp.: 234 °C, [α]_{D}²⁵ = (-) 117,5° (C=1 in Methanol) und 0.17 g (38%) (-)-**2m**HCl, Schmp.: 252 °C, [α]_{D}²⁵ = (-) 126,7° (C=1 in Methanol).

## Patentansprüche

1. Indolderivate der allgemeinen Formel **1** 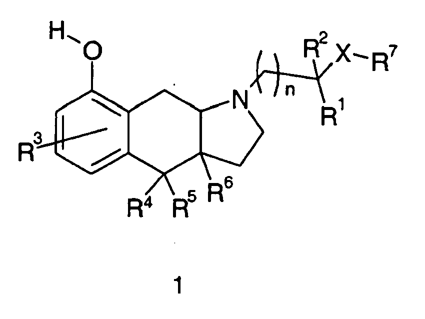 worin
X -O-, C₁-C₃-Alkylenoxy, -O-CH₂-CH₂-O- oder -O-CH₂-CH₂-NH-;
R¹ Wasserstoff, Methyl, Ethyl oder Phenyl;
R² Wasserstoff oder Methyl;
R³ Wasserstoff, F, Cl, Br, Hydroxy oder Methoxy;
R⁴ Wasserstoff, Methyl oder Ethyl;
R⁵ Wasserstoff, Methyl oder Ethyl;
R⁶ Wasserstoff, Methyl oder Ethyl;
R⁷ tert.-Butyl, Cyclohexyl, 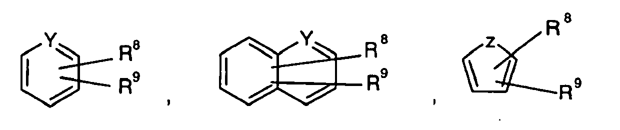
n eine ganze Zahl 0 oder 1;
Y N oder CH;
Z O, NH oder S;
R⁸ Wasserstoff, Methyl, F, Cl, Br oder Methoxy;
R⁹ Wasserstoff, Methyl, F, Cl, Br oder Methoxy
bedeuten können.

2. Verbindungen nach Anspruch 1, **dadurch gekennzeichnet, daß** sie in trans-Form vorliegen.

3. Pharmazeutische Zubereitung, **gekennzeichnet durch** einen Gehalt an einer Verbindung nach Anspruch 1 sowie deren Säureadditionssalze neben üblichen Hilfs- und Trägerstoffen.

4. Verwendung einer Verbindung nach Anspruch 1 als Arzneimittel.

5. Verwendung einer Verbindung nach Anspruch 1 zur Herstellung eines Medikamentes zur Antagonisierung der Glutamatfreisetzung.

6. Verwendung nach Anspruch 5, zur Herstellung eines Medikamentes zur Behandlung von Arrhythmien, Spasmen, kardiale und Gehirnischämien sowie neurodegenerative Erkrankungen.

7. Verwendung nach Anspruch 5, zur Herstellung eines Medikamentes zur Behandlung: Status epileptikus, Hypoglykämie, Hypoxie, Anoxie, Gehirntrauma, Gehirnoedem, Gehirn-Schlaganfall, perinatale Asphyxie, amylotrophe laterale Sklerose, Huntington's Disease, Morbus Alzheimer, Morbus Parkinson, Zyklophrenie, Hypotonie, Herzinfakt, Herzrhythmusstörungen, Angina pectoris, Schmerzen verschiedener Genese insbesondere zur Behandlung von Neuropathien.

8. Verwendung nach Anspruch 4, zur Herstellung eines Lokalanaesthetikums.

## Claims

1. Indole derivatives of general formula **1** 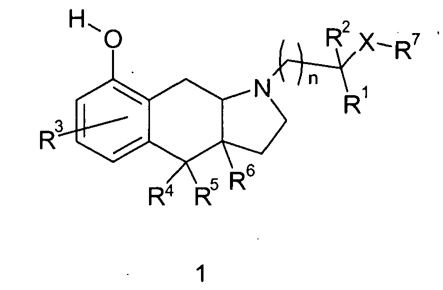 wherein
X may denote -O-, C₁-C₃-alkylenoxy, -O-CH₂-CH₂-O- or -O-CH₂-CH₂-NH-;
R¹ may denote hydrogen, methyl, ethyl or phenyl;
R² may denote hydrogen or methyl;
R³ may denote hydrogen, F, Cl, Br, hydroxy or methoxy;
R⁴ may denote hydrogen, methyl or ethyl;
R⁵ may denote hydrogen, methyl or ethyl;
R⁶ may denote hydrogen, methyl or ethyl;
R⁷ may denote tert.-butyl, cyclohexyl, 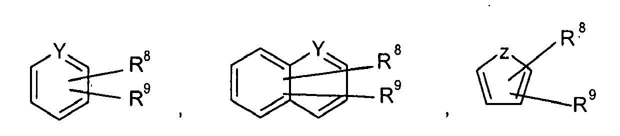
n may denote an integer 0 or 1;
Y may denote N or CH;
Z may denote O, NH or S;
R⁸ may denote hydrogen, methyl, F, Cl, Br or methoxy;
R⁹ may denote hydrogen, methyl, F, Cl, Br or methoxy.

2. Compounds according to claim 1, **characterised in that** they are present in *trans* form.

3. Pharmaceutical preparation, **characterised in that** it contains a compound according to claim 1 and the acid addition salts thereof together with conventional adjuvants and carriers.

4. Use of a compound according to claim 1 as a medicament.

5. Use of a compound according to claim 1 for the preparation of a medicament for antagonising the release of glutamate.

6. Use according to claim 5, for the preparation of a medicament for treating arrhythmias, spasms, cardiac and cerebral ischaemias and neurodegenerative diseases.

7. Use according to claim 5, for the preparation of a medicament for treating epilepsy, hypoglycaemia, hypoxia, anoxia, cerebral trauma, cerebral oedema, stroke, perinatal asphyxia, amylotropic lateral sclerosis, Huntington's disease, Alzheimer's disease, Parkinson's disease, cyclophrenia, hypotonia, cardiac infarct, heart rhythm disorders, angina pectoris, pain of various origins, particularly for treating neuropathies.

8. Use according to claim 4, for the preparation of a local anaesthetic.

## Revendications

1. Dérivés d'indole de formule générale **1** 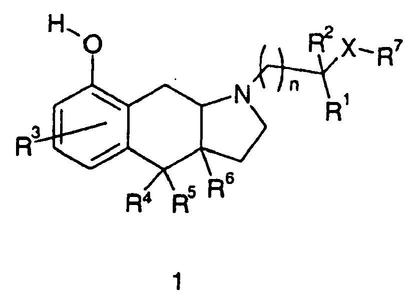 où
X peut représenter -O-, C₁-C₃-alkylénoxy, -O-CH₂-CH₂-O- ou -O-CH₂-CH₂-NH- ;
R¹ peut représenter l'hydrogène, méthyle, éthyle ou phényle ;
R² peut représenter l'hydrogène ou méthyle ;
R³ peut représenter l'hydrogène, F, CI, Br, hydroxy ou méthoxy ;
R⁴ peut représenter l'hydrogène, méthyle ou éthyle ;
R⁵ peut représenter l'hydrogène, méthyle ou éthyle ;
R⁶ peut représenter l'hydrogène, méthyle ou éthyle ;
R⁷ peut représenter tert.-butyle, cyclohexyle, 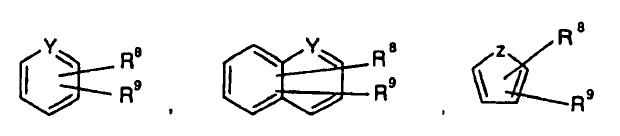
n peut représenter un nombre entier 0 ou 1 ;
Y peut représenter N ou CH ;
Z peut représenter O, NH ou S ;
R⁸ peut représenter l'hydrogène, méthyle, F, Cl, Br ou méthoxy ;
R⁹ peut représenter l'hydrogène, méthyle, F, CI, Br ou méthoxy.

2. Composés selon la revendication 1 **caractérisés en ce qu'**ils sont dans la forme trans.

3. Préparation pharmaceutique **caractérisée par** une teneur en un composé selon la revendication 1 ainsi que ses sels d'addition d'acide outre des adjuvants et supports habituels.

4. Utilisation d'un composé selon la revendication 1 comme médicament.

5. Utilisation d'un composé selon la revendication 1 pour la production d'un médicament pour l'antagonisation de la libération de glutamate.

6. Utilisation selon la revendication 5 pour la production d'un médicament pour le traitement des arythmies, des spasmes, des ischémies cardiaques et cérébrales et des maladies neurodégénératives.

7. Utilisation selon la revendication 5 pour la production d'un médicament pour le traitement : de l'épilepsie, de l'hypoglycémie, de l'hypoxie, de l'anoxie, des traumatismes cérébraux, de l'oedème cérébral, de l'attaque cérébrale, de l'asphyxie périnatale, de la sclérose latérale amyotrophique, de la maladie de Huntington, de la maladie d'Alzheimer, de la maladie de Parkinson, de la cyclothymie, de l'hypotonie, de l'infarctus cardiaque, des troubles du rythme cardiaque, de l'angine de poitrine, des douleurs de genèse diverse, en particulier pour le traitement des neuropathies.

8. Utilisation selon la revendication 4 pour la production d'un anesthésique local.
